# FASCICULE DE BREVET EUROPEEN

(11) **EP 2 524 671 B1**
(45) Date de publication et mention de la délivrance du brevet: **21.01.2015**
(21) Numéro de dépôt: 12168463.3
(22) Date de dépôt: 18.05.2012
(51) Int. Cl.: A61F 2/42, A61B 17/56

(54) **Implant articulaire d'interposition métatarso-phalangien ou métacarpo-phalangien**
Gelenkimplantat zum Einsetzen in das Zehengrundgelenk oder das Fingergrundgelenk der Mittelhand
Metatarsophalangeal or metacarpophalangeal joint implant

(30) Priorité: 19.05.2011 FR 1154343
(43) Date de publication de la demande: 21.11.2012
(73) Titulaire: Tornier, 38330 Montbonnot-Saint-Martin (FR)
(72) Inventeur: Coulange, Vincent, 69003 LYON (FR); Lizee, Emmanuel, 38330 SAINT ISMIER (FR); Hassler, Michel, 38330 SAINT ISMIER (FR); Quirantes, Gaelle, 69009 LYON (FR); Maestro, Michel, 06200 NICE (FR); Besse, Jean-Luc, 69970 CHAPONNAY (FR); Delmi, Marino, 1231 CONCHES (CH); Leemrijse, Thibaut, 1200 BRUXELLES (BE)
(74) Mandataire: Grand, Guillaume

(56) Documents cités:
- EP-A1- 1 112 753
- EP-A2- 1 637 095
- WO-A1-2006/000890
- FR-A1- 2 711 511

## Description

La présente invention concerne un implant articulaire d'interposition entre une phalange et un os associé, parmi un métatarsien et un métacarpien, d'une articulation métatarso-phalangienne ou d'une articulation métacarpo-phalangienne.

L'invention s'intéresse ainsi aux implants destinés à être mis en place à l'articulation entre un métatarsien, en particulier le premier métatarsien, ou un métatarsien et sa phalange associée, du pied ou de la main d'un être humain. L'invention s'intéresse plus particulièrement au cas où, au niveau de l'articulation métatarso-phalangienne ou métacarpo-phalangienne, l'affection de la phalange et/ou du métatarsien ou métacarpien est relativement modérée, typiquement par un hallux rigidus ou une arthrose rigidifiante, ce qui provoque une gêne et des douleurs pour le patient.

Une possibilité de traitement chirurgical consiste à fusionner l'articulation, ce qui induit sa perte irrémédiable de mobilité.

Une autre approche de traitement consiste à implanter, uniquement sur la phalange ou uniquement sur le métatarsien ou métacarpien, un composant prothétique généralement métallique. On parle alors d'hémi-arthroplastie.

Une autre approche de traitement encore consiste à implanter, sur le métatarsien ou métacarpien et sur la phalange respectivement un composant prothétique généralement métallique, séparé par un insert en polyéthylène généralement fixé sur le composant phalangien.

Une autre approche encore consiste à implanter un implant flexible généralement en silicone, implant constitué de deux tiges trouvant leur place respectivement dans la phalange d'un coté et dans le métatarsien ou métacarpien de l'autre coté.

Une approche chirurgicale alternative repose sur la mise en place, entre la phalange et le métatarsien ou métacarpien non prothésés, d'un implant articulaire d'interposition qui, lors de son implantation, n'est ancré ni vis-à-vis de la phalange, ni vis-à-vis du métatarsien ou métacarpien, mais qui est prévu pour rester stable au sein de l'articulation, en particulier pendant un temps suffisant pour régénérer les tissus osseux et cartilagineux de cette articulation. EP-A-314 593 divulgue un premier exemple d'un tel implant articulaire d'interposition, consistant en une cupule percée en son centre d'un orifice et destinée à être au moins temporairement maintenue en place entre le métatarsien ou métacarpien et la phalange grâce à une broche de fixation amovible. En pratique, la broche visant à stabiliser l'implant en son centre traverse les articulations sous jacentes (inter-phalangiennes) et traverse la peau à l'extrémité de l'orteil ou du doigt, induisant une gène post-opératoire pour le patient et des risques d'infection trouvant leur origine au passage cutané de la broche. De surcroît, eu égard à la géométrie sphérique de la cupule précitée, cette dernière se retrouve rapidement luxée ou sub-luxée dès que la broche de fixation temporaire est retirée.

De son côté, EP-A-1 637 095 a proposé d'améliorer les performances de l'implant de EP-A-314 593 en aménageant la surface distale convexe de l'implant, c'est-à-dire celle qui coopère avec la phalange, pour qu'elle bloque la rotation de l'implant sur lui-même une fois que l'implant est mis en place dans l'articulation. Pour ce faire, cette surface distale est pourvue de facettes planes ou creuses, qui rompent la symétrie de révolution de cette face distale et qui, par friction et adhérence avec la matière osseuse de la phalange, immobilise automatiquement l'implant en rotation. Cette solution, qui vise a priori à faciliter la régénération tissulaire, conduit cependant l'articulation métatarso-phalangienne ou métacarpo-phalangienne à subir un comportement très éloigné de son comportement anatomique, dans le sens où une certaine mobilité en rotation existe naturellement dans cette articulation entre le métatarsien ou métacarpien et le reste de l'orteil ou du doigt. De plus, cette solution reste sujette à des risque de dislocation, par luxation ou sub-luxation, notamment eu égard aux contraintes importantes qui sont exercées dans cette articulation lors de mouvements de la vie courante, tels que ceux de la marche.

WO-A-2006/000890 a, quant à lui, proposé un implant d'interposition sans tige d'ancrage osseux, pour les articulations du doigt. Le corps de l'implant présente une face proximale concave, en particulier sphérique, et, à l'opposé, une face distale qui est soit concave elle aussi, en particulier sphérique ou cylindrique, soit plane ou convexe, sans autre précision. En service, la stabilité de cet implant est précaire, ce qui explique pourquoi il y est prévu un trou traversant dans lequel est enfilé un fil de suture lié aux structures anatomiques environnantes.

Le but de la présente invention est de proposer un implant articulaire qui, tout en respectant au mieux l'anatomie de l'articulation métatarso-phalangienne, puisse être interposé de manière stable entre le métatarsien et la phalange.

A cet effet, l'invention a pour objet un implant articulaire d'interposition entre une phalange et un os associé, parmi un métatarsien et un métacarpien, d'une articulation métatarso-phalangienne ou d'une articulation métacarpo-phalangienne, tel que défini à la revendication 1.

Une des idées à la base de l'invention est de chercher à augmenter la stabilité de l'implant au sein d'une articulation métatarso-phalangienne ou d'une articulation métacarpo-phalangienne, sans pour autant l'immobiliser complètement, notamment en lui laissant une certaine mobilité en rotation sur lui-même. Suivant l'invention, la face distale de l'implant, qui est en forme globale de pointe saillante dans la perspective de coopérer en service avec la phalange, présente une surface principale spécifique de géométrie remarquable. D'une part, en coupe proximo-distale, c'est-à-dire en n'importe quel plan de coupe contenant l'axe proximo-distal de l'implant, cette surface présente un profil concave ayant pour effet d'auto-stabiliser l'implant, c'est-à-dire de le maintenir en place en s'opposant à tout basculement de l'implant autour d'un axe géométrique transversal à l'axe proximo-distal de l'implant. D'autre part, en coupe perpendiculaire à l'axe proximo-distal, c'est-à-dire en n'importe quel plan perpendiculaire le long de cet axe proximo-distal, cette surface présente un contour fermé continûment incurvé, autrement dit dépourvu de toute portion anguleuse ou plate, ce qui a pour effet de ne pas immobiliser l'implant en rotation sur lui-même autour de l'axe proximo-distal. Ainsi, les capacités cinématiques et mécaniques de l'implant conforme à l'invention sont parfaitement adaptées aux besoins anatomiques d'une articulation métatarso-phalangienne ou d'une articulation métacarpo-phalangienne. De plus, la géométrie globalement pointue de la face distale de l'implant permet avantageusement de conserver un maximum de la matière osseuse phalangienne présente.

En pratique, l'implant suivant l'invention se décline en une gamme de plusieurs implants présentant des encombrements respectifs différents, les dimensions proximo-distales respectives de ces implants étant avantageusement liées à leurs dimensions transversales maximales respectives. Dans tous les cas, la taille de l'implant permet avantageusement de stabiliser immédiatement son corps par la seule contrainte de l'enveloppe capsulaire et ligamentaire autour de l'articulation métatarso-phalangienne ou métacarpo-phalangienne, sans pour autant que ce corps subisse une hyper-contrainte puisqu'il doit conserver une certaine mobilité, notamment en rotation, afin que sa position, notamment angulaire, s'adapte en fonction des contraintes de l'enveloppe articulaire, liées aux différents mouvements possibles de l'articulation.

Des caractéristiques additionnelles avantageuses de l'implant conforme à l'invention, prises isolément ou selon toutes les combinaisons techniquement possibles, sont spécifiées aux revendications dépendantes 2 à 11.

L'invention sera mieux comprise à la lecture de la description qui va suivre, donnée uniquement à titre d'exemple et faite en se référant aux dessins sur lesquels :
- les figures 1 et 2 sont des vues en perspective, sous des angles de vue différents, d'un premier mode de réalisation d'un implant conforme à l'invention ;
- la figure 3 est une vue en élévation selon la flèche III de la figure 1 ;
- la figure 4 est une vue en élévation suivant la flèche IV de la figure 3 ;
- la figure 5 est une coupe selon la ligne V-V de la figure 3 ;
- la figure 6 est une coupe dans le plan indiqué par la ligne VI-VI de la figure 3, illustrant l'implant en place au sein d'une articulation métatarso-phalangienne ;
- les figures 7 et 8 sont des vues en perspective, sous des angles de vue différents, d'un second mode de réalisation d'un implant conforme à l'invention ;
- la figure 9 est une vue en élévation suivant la flèche IX de la figure 8 ;
- les figures 10 et 11 sont des coupes respectivement selon les lignes X-X et XI-XI de la figure 9 ; et
- la figure 12 est une vue analogue à la figure 6, montrant l'implant des figures 7 à 11 en place au sein d'une articulation métatarso-phalangienne.

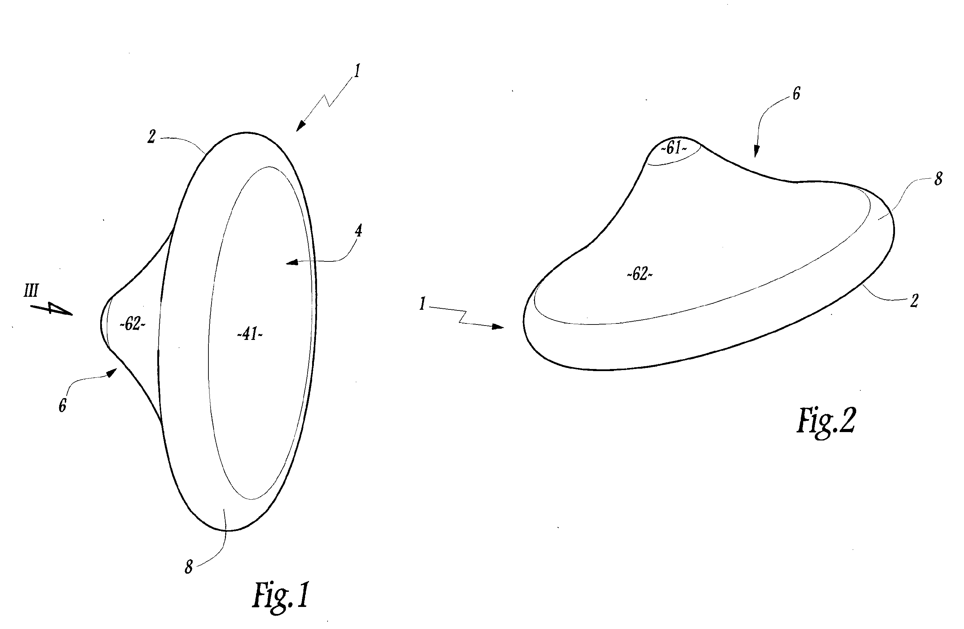

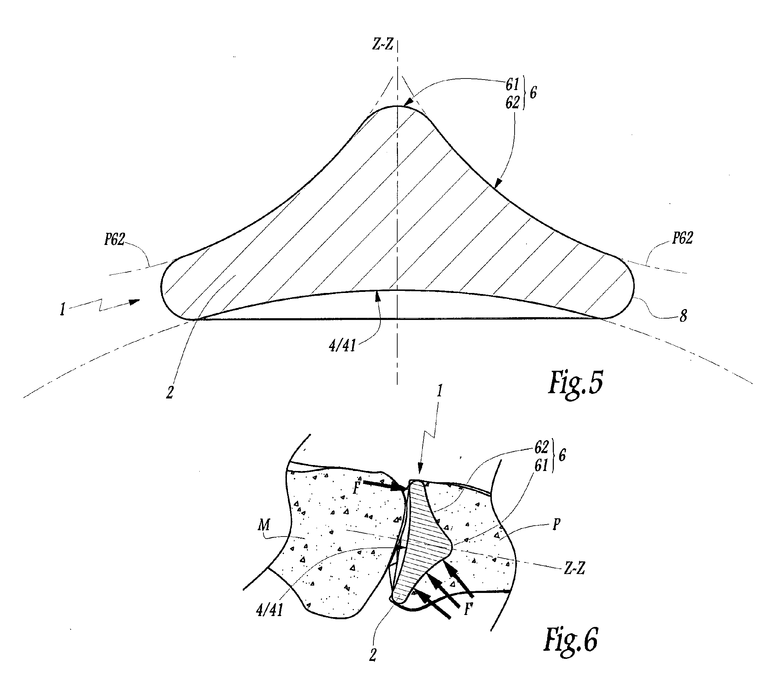

Sur les figures 1 à 5 est représenté un implant articulaire 1 adapté pour être implanté de manière interposée entre un métatarsien M, en particulier le premier métatarsien, et la phalange P associée à ce métatarsien, comme représenté sur la figure 6, en vue de rétablir l'articulation métatarso-phalangienne entre les os précités du pied d'un patient.

Dans tout le présent document, lorsqu'un terme ayant un sens anatomique usuel est utilisé pour décrire l'implant 1, l'implant est considéré dans sa configuration d'implantation au sein de l'articulation métatarso-phalangienne.

L'implant 1 comporte un corps 2, qui va être décrit en détail ci-après et qui, globalement, présente la forme d'un chapeau de champignon ou d'un « chapeau chinois ». Le corps 2 s'étend le long et autour d'un axe central proximo-distal Z-Z qui, en configuration d'implantation de l'implant 1, s'étend sensiblement suivant la direction longitudinale de la phalange P.

Le corps 2 présente ainsi deux faces principales, opposées suivant l'axe Z-Z, à savoir une face proximale 4 et une face distale 6. Ces faces 4 et 6 sont séparées l'une de l'autre par l'épaisseur, selon l'axe Z-Z, du corps 2, en étant reliées l'une à l'autre par une bordure périphérique 8 du corps 2. Dans le mode de réalisation considéré sur les figures, la bordure 8 présente une faible épaisseur proximo-distale et est émoussée, raccordant les faces 4 et 6 directement l'une à l'autre en quelque sorte. A titre de variante non représentée, la bordure 8 peut présenter une surface périphérique cylindrique, centrée sur l'axe Z-Z.

Dans la forme de réalisation envisagée sur les figures 1 à 6, l'implant 1 n'est constitué que du corps 2, dans le sens où l'implant ne comporte aucun autre composant, notamment aucun composant de fixation osseuse qui permettrait d'ancrer fixement le corps 2 dans le métatarsien M et/ou dans la phalange P. Dans ce cas, le corps 2 constitue un implant exclusivement d'interposition de manière que ce corps présente, après implantation entre le métatarsien M et la phalange P, une certaine liberté de mouvements, sa position s'adaptant aux contraintes qui lui sont appliquées, en fonction des mouvements de l'articulation métatarso-phalangienne.

Dans cette optique, le corps 2 est avantageusement réalisé d'un seul tenant en céramique, notamment en pyrocarbone, autrement appelé carbone pyrolytique, ce qui confère à ce corps une bonne biocompatibilité, une haute résistance mécanique et une élasticité proche de l'os, tout en limitant, voire quasi annulant l'usure du métatarsien M et de la phalange P. A titre de variante, d'autres matériaux peuvent être utilisés, notamment un alliage métallique à base de chrome et de cobalt, un alliage métallique à base de titane, un alliage métallique à base d'acier inoxydable, une matière plastique, notamment le polyéthylène et le PEEK, etc.

La face proximale 4 du corps 2 s'apparente, globalement, à une portion de sphère. Plus précisément, sur cette face 4, le corps 2 délimite une surface concave 41 qui, dans le mode de réalisation représenté sur les figures 1 à 6, occupe la totalité de la face 4. En service, la surface concave 41 est destinée à s'articuler directement contre le métatarsien M, plus précisément contre la tête distale de ce métatarsien, comme représenté sur la figure 6. A cette fin, dans le mode de réalisation des figures 1 à 6, la surface concave 41 est sphérique : géométriquement, elle est constituée d'une calotte sphérique qui est centrée sur l'axe Z-Z dans le sens où cet axe constitue un axe de révolution pour la surface en calotte 41.

Comme évoqué plus haut, la face distale 6 du corps 2 présente, globalement, une forme en pointe. Cette forme globale en pointe est saillante par rapport au reste du corps 2, dans le sens où, à l'inverse, la face proximale 4 est en creux en égard à la surface concave 41. Plus précisément, dans la région centrale de la face 6, le corps 2 délimite une surface convexe 61, qui est centrée sur l'axe Z-Z et qui peut être qualifiée de surface de sommet dans le sens où cette surface 61 est située, suivant l'axe proximo-distal Z-Z, au niveau de l'extrémité distale du corps 2. Cela revient à dire que la forme en pointe est sensiblement centrée sur l'axe Z-Z et a pour sommet, c'est-à-dire pour région culminante de la face distale 6, la surface 61 précitée.

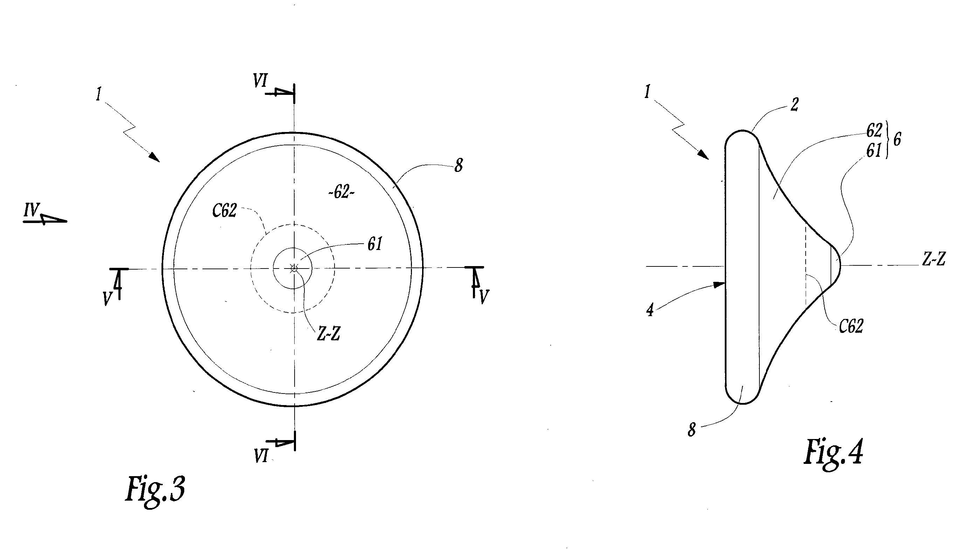

Sur sa face distale 6, le corps 2 délimite en outre une surface élaborée 62 qui occupe la majeure partie de la face 6, en reliant, suivant toute la périphérie de cette face, la surface de sommet 61 à la bordure périphérique 8, comme bien visible sur la figure 3. Cette surface 62 s'étend ainsi tout autour de l'axe Z-Z, sans être intersectée par cet axe. Dans le mode de réalisation des figures 1 à 6, la surface 62 est une surface de révolution qui est centrée sur l'axe Z-Z et dont la génératrice est un segment de courbe concave, notamment un arc de cercle concave ou, à titre de variantes non représentées, un arc d'ellipse concave ou une arc concave pseudo-circulaire ou pseudo-elliptique : cela revient à dire que, d'une part, en un quelconque plan de coupe perpendiculaire à l'axe Z-Z, la surface 62 présente un contour fermé circulaire, centré sur l'axe Z-Z, comme indiqué, à titre d'exemple, par le contour en pointillés C62 sur les figures 3 et 4, et, d'autre part, dans n'importe quel plan de coupe contenant l'axe Z-Z, la surface 62 présente un profil concave constitué de deux arcs concaves, symétriques l'un de l'autre par rapport à l'axe Z-Z, comme indiqué, à titre d'exemple, par les deux arcs en traits mixtes P62 sur la figure 5.

L'intérêt de l'agencement de la face distale 6 du corps 2 de l'implant 1, notamment avec sa surface élaborée 62, va être mieux compris ci-dessous, dans le cadre de la description d'un exemple d'une intervention chirurgicale visant à mettre en place l'implant au niveau de l'articulation entre le métatarsien M et la phalange P.

Dans un premier temps opératoire, un chirurgien accède à la zone articulaire entre le métatarsien M et la phalange P, notamment via une voie d'abord dorsale ou médiale pour accéder à l'espace interosseux entre le métatarsien et la phalange. La capsule articulaire et les ligaments entourant cette zone articulaire sont soit respectés, en étant écartés, soit partiellement incisés, étant entendu que, dans ce dernier cas, la capsule et les ligaments seront reconstruits en fin d'intervention.

Le chirurgien écarte ensuite l'un de l'autre le métatarsien M et la phalange P afin d'élargir l'espace interosseux, notamment en exerçant un effort de traction suivant la direction longitudinale de l'orteil. Le cas échéant, le chirurgien peut alors préparer l'une et/ou l'autre des surfaces osseuses en regard délimitées par la tête du métatarsien M et la phalange P, de manière à les conformer de façon complémentaire respectivement aux surfaces 41 et 62 du corps 2 de l'implant 1. Cette étape est avantageusement facilitée par une subluxation partielle de la phalange par rapport au métatarsien. En pratique, cette étape de préparation des surfaces osseuses peut ne pas être nécessaire, selon l'état effectif de ces surfaces : en effet, à l'état naturel non affecté, on notera que les extrémités en regard du métatarsien M et de la phalange P présentent des géométries de surface respectives proches de surfaces complémentaires aux surfaces 41 et 62, de sorte que les réaménagements osseux, tels que des découpes et des fraisages, réalisés sur ces extrémités osseuses peuvent être minimes pour adapter la surface de ces os aux surfaces 41 et 62. D'ailleurs, en pratique, de tels réaménagements sont généralement minimes, voire inutiles, sur le métatarsien, tandis que des réaménagements plus importants doivent a priori être réalisés sur la phalange. Dans tous les cas, la préparation des extrémités en regard du métatarsien M et de la phalange P n'entame pas profondément ces os, en restant au niveau de leur couche cartilagineuse ou de leur couche corticale osseuse. Autrement dit, le stock osseux initialement présent au niveau de l'articulation métatarso-phalangienne n'est que peu, voire pas entamé.

Dans un second temps opératoire, tout en maintenant la traction sur l'articulation métatarso-phalangienne selon la direction longitudinale de l'orteil, le chirurgien positionne l'implant 1 dans l'espace interosseux séparant le métatarsien et la phalange, en interposant le corps 2 entre ces deux os de manière que la surface 4 est tournée vers le métatarsien tandis que la surface 6 est tournée vers la phalange. A ce stade, on remarquera que, dans la mesure où les surfaces 41 et 62 sont, dans le mode de réalisation des figures 1 à 5, des surfaces de révolution, toutes deux centrées sur l'axe Z-Z, le positionnement angulaire, autour de cet axe Z-Z, de l'implant 1 mis en place entre le métatarsien M et la phalange P n'a aucune importance.

Avantageusement, l'engagement de la surface 62 avec la surface osseuse sensiblement complémentaire de la phalange P est facilité par la surface de sommet 61, dans le sens où cette surface 61 peut s'appuyer et glisser contre la surface osseuse précitée, jusqu'à centrer le corps 2 vis-à-vis de la phalange P, c'est-à-dire à faire sensiblement coïncider l'axe Z-Z avec l'axe du canal médullaire de la phalange. Cet effet d'auto-centrage est d'autant facilité que, d'un côté, la surface au sommet 61 est convexe tandis que, de l'autre côté, la surface osseuse précitée de la phalange P est à profil convexe convergent, comme visible sur la figure 6.

Une fois que l'implant 1 est ainsi placé entre le métatarsien M et la phalange P, la traction axiale précitée est relâchée, de sorte que la capsule articulaire et les ligaments entourant l'articulation métatarso-phalangienne rapprochent l'un de l'autre le métatarsien et la phalange. Le corps 2 de l'implant 1 se trouve alors retenu entre le métatarsien et la phalange, sous la contrainte de la capsule et des ligaments, autrement dit sous une contrainte dont la résultante est sensiblement alignée avec l'axe Z-Z. Le chirurgien peut ensuite refermer les tissus mous autour de l'articulation, le cas échéant par reconstruction.

L'articulation métatarso-phalangienne ainsi munie de l'implant 1 retrouve un comportement cinématique proche, voire quasi identique au comportement anatomique d'origine de cette articulation. En effet, comme représenté sur la figure 6, le métatarsien M s'articule alors contre la surface 41, essentiellement à la façon d'une rotule, tandis que, dans le même temps, l'implant 1 est libre de tourner sur lui-même autour de l'axe Z-Z par rapport à la phalange P, par coopération dynamique entre la surface 62 et la surface osseuse complémentaire de la phalange. Bien entendu, en pratique, seuls des débattements rotatifs de quelques degrés, voire moins, sont constatés entre l'implant 1 et la phalange P, ces débattements apparaissant lors des déformations que subit l'orteil au cours de la marche. Cet effet de mobilité en rotation est évidemment lié au contour circulaire de la surface 62. De plus, également par coopération dynamique entre la surface 62 et la surface osseuse complémentaire de la phalange P, l'implant 1 se trouve autostabilisé entre le métatarsien M et la phalange, dans le sens où, lorsqu'une contrainte est appliquée à l'implant 1, tendant à le luxer ou le subluxer, typiquement sous l'action d'une force référencée F sur la figure 6, des contraintes de résultante opposée apparaissent sur la portion de la surface 62, diamétralement opposée à la zone d'application de la contrainte précitée, comme indiqué schématiquement par les forces F' sur la figure 6. Cet effet d'auto-stabilisation de l'implant 1 est évidemment lié au profil concave de la surface 62.

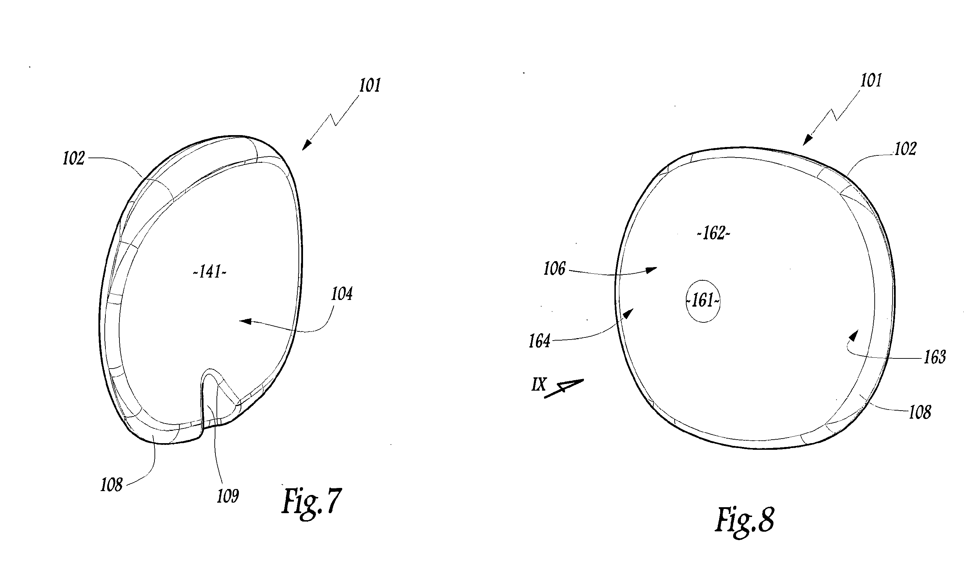

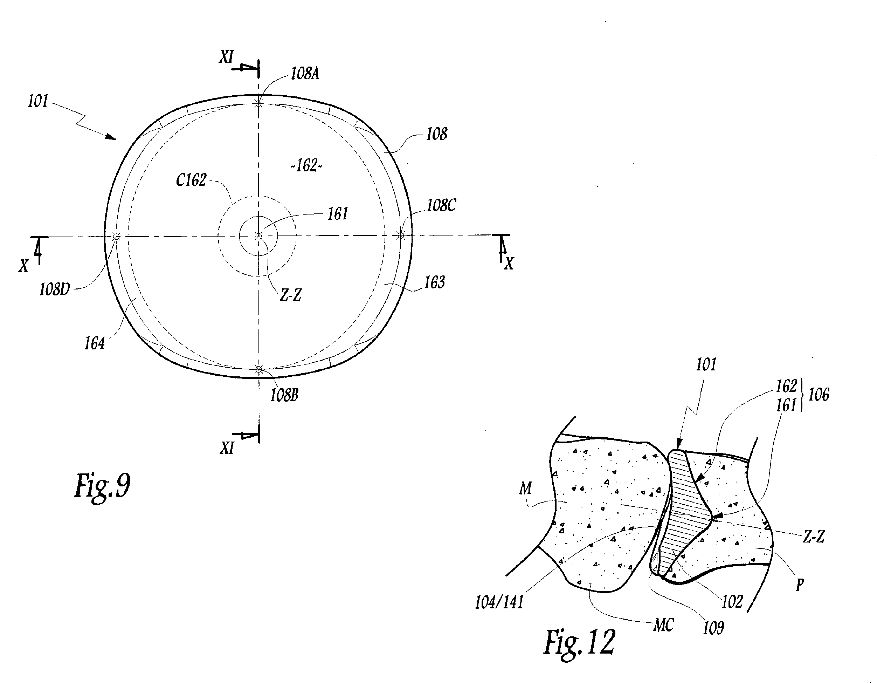

Sur les figures 7 à 12 est représentée une variante de réalisation de l'implant 1, référencée 101. Cet implant 101 comporte un corps 102 similaire au corps 2 de l'implant 1, dans le sens où, sur sa face proximale 104, le corps 102 délimite une surface concave 141 qui est fonctionnellement similaire à la surface 41 de l'implant 1 et, sur sa face distale 106, le corps 102 délimite des surfaces 161 et 162, qui sont fonctionnellement similaires aux surfaces 61 et 62 de l'implant 1, les faces proximale 104 et distale 106 étant reliées l'une à l'autre par une bordure périphérique 108 du corps 102. L'implant 101 peut être utilisé en lieu et place de l'implant 1, en vue d'être interposé entre le métatarsien M et la phalange P pour rétablir l'articulation métatarso-phalangienne, comme représenté sur la figure 12. Toutefois, l'implant 101 se distingue de l'implant 1 par trois aspects, qui vont être successivement détaillés ci-après.

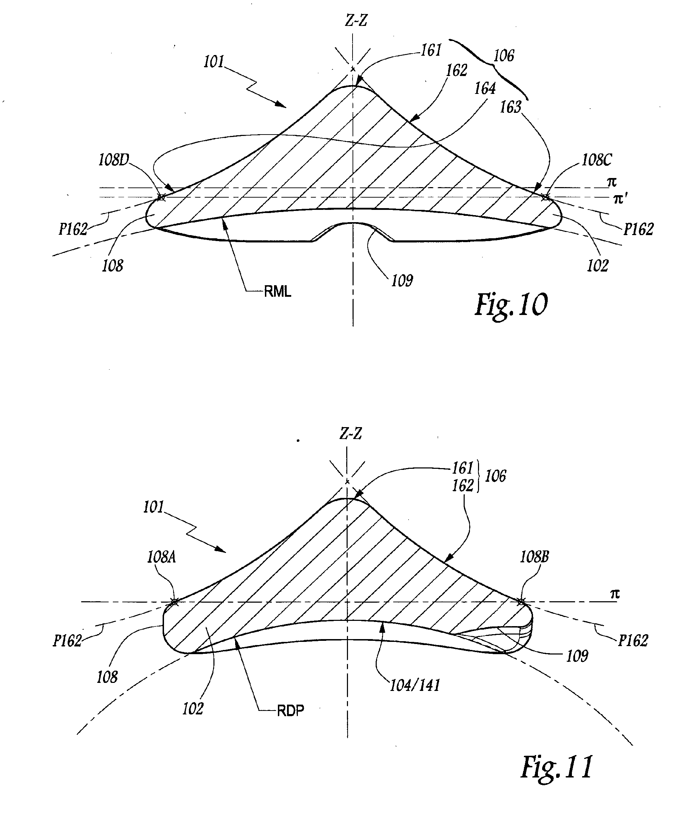

Selon un premier aspect distinctif, la surface 162 est relié directement à la bordure périphérique 108 non pas sur toute la périphérie de la face distale 106 du corps 102, comme l'est la surface 62 avec la bordure 8, mais seulement en deux points 108A et 108B diamétralement opposés, par lesquels passe la ligne de coupe XI-XI. En effet, les deux portions de la surface 162, opposées selon la ligne de coupe X-X, sont reliées à la bordure 108 par des surfaces respectives 163 et 164 qui, comme bien visible aux figures 9 et 10, s'étendent dans le prolongement de la surface 162. Ainsi, dans la partie axiale de la face 106, allant de l'extrémité distale du corps 102 au plan géométrique référencé π sur les figures 10 et 11, c'est-à-dire le plan perpendiculaire à l'axe Z-Z et passant par les deux points 108A et 108B, cette face 106 présente exclusivement la surface de sommet 161 et la surface 162 et correspond ainsi géométriquement à la face 6 de l'implant 1 : en particulier, la surface 162 est géométriquement identique à la surface 62, en présentant notamment un contour transversal circulaire, comme, à titre d'exemple, le contour en pointillés C162 sur la figure 9, voire en correspondant à une surface de révolution centrée sur l'axe Z-Z. Puis, dans la partie axiale de la face 106, comprise entre le plan précité π et le plan géométrique référencé π' sur la figure 10, c'est-à-dire le plan perpendiculaire à l'axe Z-Z et passant par les deux points 108C et 108D d'intersection, sur la figure 9, entre la bordure 108 et la ligne de coupe X-X, la face 106 présente exclusivement les surfaces 163 et 164 qui, en quelque sorte, prolongent localement la surface 162. En projection dans un plan perpendiculaire à l'axe Z-Z, comme bien visible à la figure 9, la bordure 108 présente un contour sensiblement elliptique, avec le segment reliant les points 108C et 108D qui correspond au grand axe de ce contour elliptique, tandis que le segment reliant les points 108A et 108B correspond au petit axe de ce contour elliptique. Ainsi, en projection dans un plan perpendiculaire à l'axe Z-Z, chacune des surfaces 163 et 164 présente globalement la forme d'un croissant de lune, avec un contour intérieur consistant en un arc de cercle centré sur l'axe Z-Z et reliant les points 1 08A et 108B, tandis que son contour extérieur est un arc d'ellipse, également centré sur l'axe Z-Z et reliant les points 108A et 108B. La réunion des contours intérieurs des deux croissants de lune précités constitue le contour périphérique circulaire de la surface 162, tandis que la réunion des contours extérieurs des deux croissants de lune précités constitue le contour périphérique elliptique de la bordure 108.

Bien entendu, le fait que la surface 162 soit, en quelque sorte, prolongée localement par les surfaces 163 et 164 est parfaitement compatible, du point de vue géométrique, avec le fait que, en coupe axiale, la surface 162 présente un profil concave. D'ailleurs, dans le mode de réalisation représenté sur les figures 7 à 12, le profil de la surface 162 est de la même nature géométrique que le profil de la surface 162 de l'implant 1, à savoir que, comme indique, à titre d'exemple, par les deux arcs de cercle en traits mixtes P162 sur les figures 10 et 11, le profil concave de la surface 162 est circulaire. De plus, comme expliqué plus haut, les surfaces 163 et 164 présentent, elles aussi, en coupe axiale, un profil concave, qui s'inscrit dans le prolongement continu du profil concave de la surface 162, comme bien visible sur la figure 10.

On notera que, eu égard à la présence des surfaces 163 et 164, la surface d'ensemble correspondant à la réunion des surfaces 162, 163 et 164 ne peut pas être une surface de révolution. Toutefois, cette surface d'ensemble peut être considérée comme une surface de révolution à laquelle on a enlevé, dans sa partie d'extrémité axiale opposée à la surface de sommet 161, deux portions opposées selon la ligne de coupe XI-XI, par intersection avec une surface ellipsoïde. Cette présentation de la surface d'ensemble précitée permet de bien comprendre l'intérêt de cette surface d'ensemble, à savoir que, d'une part, comme pour l'implant 1, l'implant 101 est libre de tourner sur lui-même autour de l'axe Z-Z par rapport à la phalange P, par coopération dynamique entre la surface 162 et la surface osseuse complémentaire de la phalange, et, d'autre part, les surfaces 163 et 164 assurent une meilleure assise de l'implant 101 sur l'extrémité proximale de la phalange P, autrement dit sur l'extrémité de la phalange en regard du métatarsien M, dont la surface osseuse présente une section transversale sensiblement elliptique, avec un grand diamètre s'étendant suivant une direction médio-latérale tandis que son petit diamètre s'étend suivant une direction dorso-plantaire. Ainsi, en mettant en place l'implant 101 de sorte que la ligne de coupe X-X s'étend suivant une direction médio-latérale à la phalange P, les surfaces 163 et 164 s'étendent respectivement dans le prolongement de la portion médiale et dans le prolongement de la portion latérale de la surface 162 et se trouvent efficacement assises sur la base proximale de la phalange, permettant ainsi de mieux répartir les contraintes entre l'implant et la phalange, tout en autorisant la mobilité rotative de l'implant sur lui-même autour de l'axe Z-Z, par coopération entre la surface 162 et la partie courante de la phalange. Bien entendu, selon la conformation osseuse de la phalange P, en particulier de son extrémité proximale, il n'est pas exclu que les surfaces 163 et 164 limitent la course rotative maximale de l'implant vis-à- vis de la phalange, étant cependant rappelé que, en pratique, en configuration d'implantation, la mobilité rotative de l'implant 101 s'apparente, dans tous les cas, à de petits débattements angulaires, qui sont permis et guidés par la surface 162, sans être bloquée par les surfaces 163 et 164.

Sur la base des considérations justes précédentes, on comprend qu'une variante, non représentée, consiste à ce que la surface 62 ou la surface 162 ne présente pas, en coupe transversale à l'axe Z-Z, un contour rigoureusement circulaire, mais un contour fermé ellipsoïdal : en n'importe quel plan de coupe perpendiculaire à l'axe Z-Z, la surface 62 ou 162 présente alors un contour fermé qui est continûment incurvé, c'est-à-dire dépourvu d'angulation ou de plat, ce qui autorise une certaine mobilité rotative de l'implant 1 ou 101 sur lui-même, autour de l'axe Z-Z, après implantation de cet implant sur la phalange P, ne serait-ce que sous la forme de petits débattements angulaires, non bloqués par le contour continûment incurvé précité. Comparativement aux implants 1 et 101, on comprend que l'implant selon cette variante présente une moins grande mobilité rotative sur lui-même autour de l'axe Z-Z, mais le contour ellipsoïdal précité présente l'avantage, dans la partie d'extrémité axiale de la surface 62 ou 162, opposée à la surface de sommet 61 ou 161, de mieux respecter l'anatomie de l'extrémité proximale de la phalange P, comme expliqué plus haut.

Selon un second aspect distinctif de l'implant 101 par rapport à l'implant 1, la surface concave 141 de la face proximale 4 du corps 102 n'est pas rigoureusement une calotte sphérique, dans le sens où la courbure de cette surface 141 n'est pas constante. Plus précisément, la surface 141 présente :
- dans le plan de la figure 10, autrement dit en coupe axiale médio-latérale, un rayon de courbure noté RML, et
- dans le plan de la figure 11, c'est-à-dire en coupe axiale dorso-plantaire, un rayon de courbure qui est noté RDP et qui est strictement inférieur au rayon de courbure RML.

De cette façon, la surface concave 141 présente une géométrie mieux ajustée complémentairement à la surface articulaire anatomique de la tête du métatarsien M. Il en résulte que, dans la configuration d'implantation de l'implant 101, la surface concave 141 s'articule contre le métatarsien M avec une cinématique quasi-identique à la cinématique naturelle entre des os sains.

Enfin, selon un troisième aspect distinctif de l'implant 101 vis-à-vis de l'implant 1, le corps 102 délimite, sur sa face proximale 104, une échancrure 109 qui, comme bien visible sur les figures 7 et 11, est située dans la portion plantaire de la face 104, en s'étendant suivant une direction dorso-plantaire de manière à déboucher sur une portion plantaire de la bordure périphérique 108 du corps 102. Cette échancrure 109 est ainsi positionnée et dimensionnée pour recevoir, au moins lors de certains mouvements de l'orteil lors de la marche, la crête sésamoïdienne MC présente dans la portion plantaire de la tête du métatarsien M. Autrement dit, grâce à l'échancrure 109, on limite les risques d'interférence entre la portion plantaire de la bordure 108 et cette crête métatarsienne MC.

Bien entendu, on comprendra que chacun des trois aspects distinctifs de l'implant 101 vis-à-vis de l'implant 1, qui ont été présentés successivement ci-dessus, peut être mis en oeuvre indépendamment des deux autres. Par exemple, une variante non représentée consiste en l'implant 1 muni de l'échancrure 109. De même, un autre exemple de variante non représentée consiste en l'implant 101 présentant, en remplacement de sa surface concave 141, la surface concave 41 de l'implant 1, avec ou sans la présence de l'échancrure 109.

Divers aménagements et variantes aux implants 1 et 101, ainsi qu'à leur méthode d'implantation sont par ailleurs envisageables. Par exemple, comme évoqué plus haut, les implants 1 et 101 peuvent être prévus pour être implantés entre un métatarsien autre que le premier métatarsien et sa phalange associée du pied d'un patient. Une autre application des implants 1 et 101 concerne les articulations métacarpo-phalangiennes de la main d'un patient, les implants 1 et 101 étant alors prévus pour être implantés entre un métacarpien et sa phalange associée.

## Revendications

1. Implant articulaire (1 ; 101) d'interposition entre une phalange (P) et un os associé, parmi un métatarsien et un métacarpien, d'une articulation métatarso-phalangienne ou d'une articulation métacarpo-phalangienne,
comportant un corps (2, 102) qui présente, à l'opposé l'une de l'autre suivant un axe proximo-distal (Z-Z), des faces proximale (4 ; 104) et distale (6 ; 106) destinées à être tournées respectivement vers l'os associé (M) et vers la phalange (P), le corps délimitant, sur sa face proximale, une surface concave (41 ; 141) d'articulation avec l'os associé,
**caractérisé en ce que** la face distale (6 ; 106) du corps (2 ; 102) présente globalement une forme en pointe délimitant une surface (62 ; 162) de coopération dynamique avec la phalange (P), qui s'étend tout autour de l'axe proximo-distal (Z-Z), qui présente, en coupe perpendiculaire à l'axe proximo-distal, un contour fermé continûment incurvé (C62 ; C162), et qui présente, en coupe axiale, un profil concave (P62 ; P162).

2. Implant suivant la revendication 1, **caractérisé en ce que** le corps (2 ; 102) présente, suivant l'axe proximo-distal (Z-Z), une extrémité distale au niveau de laquelle est située une surface de sommet (61 ; 161), qui est délimitée par une région, centrée sur l'axe proximo-distal, de la face distale (6 ; 106), et depuis laquelle s'étend la surface (62 ; 162) de coopération dynamique avec la phalange (P).

3. Implant suivant l'une des revendications 1 ou 2, **caractérisé en ce que** ledit contour fermé (C62 ; C162) de la surface (62 ; 162) de coopération dynamique avec la phalange (P) est un cercle centré sur l'axe proximo-distal (Z-Z).

4. Implant suivant la revendication 3, **caractérisé en ce que** la surface (62 ; 162) de coopération dynamique avec la phalange (P) est une surface de révolution, qui est centrée sur l'axe proximo-distal (Z-Z) et dont la génératrice correspond à un segment du profil concave (P62 ; P162).

5. Implant suivant l'une quelconque des revendications précédentes, **caractérisé en ce que**, sur sa face distale (106), le corps (102) délimite au moins une surface (163, 164) d'assise sur la phalange (P), qui relie une portion seulement de la surface de coopération dynamique (162), en particulier sa portion médiale ou sa portion latérale, à la bordure périphérique (108) du corps, en s'étendant dans le prolongement de la surface dynamique.

6. Implant suivant l'une des revendications 1 ou 2, **caractérisé en ce que** ledit contour fermé de la surface de coopération dynamique avec la phalange (P) est une ellipsoïde sensiblement centrée sur l'axe proximo-distal (Z-Z).

7. Implant suivant l'une quelconque des revendications précédentes, **caractérisé en ce que** ledit profil concave (P62 ; P162) de la surface (62 ; 162) de coopération dynamique avec la phalange (P) est sensiblement circulaire.

8. Implant suivant l'une quelconque des revendications précédentes, **caractérisé en ce que** la surface concave (41) d'articulation avec l'os associé (M) est une calotte sphérique, centrée sur l'axe proximo-distal (Z-Z).

9. Implant suivant l'une quelconque des revendications 1 à 7, **caractérisé en ce que** la surface concave (141) d'articulation avec l'os associé (M) présente, en coupe dans un plan axial dorso-plantaire ou dorso-palmaire, un rayon de courbure (RDP) inférieur à son rayon de courbure (RML) dans un plan axial médio-latéral.

10. Implant suivant l'une quelconque des revendications précédentes, **caractérisé en ce que** le corps (102) délimite, sur sa face proximale (104), une échancrure (109) de réception d'une crête osseuse de l'os associé, en particulier de la crête sésamoïdienne (MC) du métatarsien (M), laquelle échancrure débouche sur une portion plantaire ou palmaire de la bordure périphérique (108) du corps.

11. Implant suivant l'une quelconque des revendications précédentes, **caractérisé en ce que** l'implant (1 ; 101) est constitué exclusivement du corps (2 ; 102) réalisé d'une seule pièce.

## Patentansprüche

1. Gelenkimplantat (1, 101) zum Anordnen zwischen einer Phalanx (P) und einem zugeordneten Knochen aus einem Metatarsus und einem Metacarpus für ein Metatarsus-Phalanx-Gelenk oder ein Metacarpus-Phalanx-Gelenk,
aufweisend einen Körper (2, 102), der einander entgegengesetzt entlang einer proximodistalen Achse (Z-Z) eine proximale (4, 104) und eine distale (6, 106) Seite aufweist, die vorgesehen sind, um jeweils zu dem zugeordneten Knochen (M) und zu der Phalanx (P) hin gedreht zu sein, wobei der Körper auf seiner proximalen Seite eine konkave Fläche (41, 141) für eine Gelenkverbindung mit dem zugeordneten Knochen begrenzt, **dadurch gekennzeichnet, dass** die distale Seite (6, 106) des Körpers (2, 102) insgesamt eine spitz zulaufende Form hat, die eine Fläche (62, 162) zum dynamischen Zusammenwirken mit der Phalanx (P) begrenzt, die sich rund um die proximodistale Achse (Z-Z) erstreckt, die im Schnitt senkrecht zu der proximodistalen Achse einen durchgehend gekrümmten geschlossenen Umriss (C62, C162) aufweist, und die im Axialschnitt ein konkaves Profil (P62, P162) aufweist.

2. Implantat gemäß Anspruch 1, **dadurch gekennzeichnet, dass** der Körper (2, 102) entlang der proximodistalen Achse (Z-Z) ein distales Ende aufweist, auf dessen Höhe eine Scheitelfläche (61, 161) angeordnet ist, die durch einen auf der proximodistalen Achse zentrierten Bereich der distalen Seite (6, 106) begrenzt ist und von der aus sich die Fläche (62, 162) zum dynamischen Zusammenwirken mit der Phalanx (P) erstreckt.

3. Implantat gemäß einem der Ansprüche 1 oder 2, **dadurch gekennzeichnet, dass** der geschlossene Umriss (C62, C162) der Fläche (62, 162) zum dynamischen Zusammenwirken mit der Phalanx (P) ein auf der proximodistalen Achse (Z-Z) zentrierter Kreis ist.

4. Implantat gemäß Anspruch 3, **dadurch gekennzeichnet, dass** die Fläche (62, 162) zum dynamischen Zusammenwirken mit der Phalanx (P) eine Umdrehungsfläche ist, die auf der proximodistalen Achse (Z-Z) zentriert ist und deren Mantellinie mit einem Segment des konkaven Profils (P62, P162) korrespondiert.

5. Implantat gemäß irgendeinem der vorhergehenden Ansprüche, **dadurch gekennzeichnet, dass** der Körper (102) auf seiner distalen Seite (106) mindestens eine Auflagefläche (163, 164) für die Phalanx (P) begrenzt, die einen Abschnitt lediglich der Fläche zum dynamischen Zusammenwirken (162), insbesondere ihren medialen Abschnitt oder ihren lateralen Abschnitt mit dem Umfangsrand (108) des Körpers verbindet, wobei sie sich in der Verlängerung der dynamischen Fläche erstreckt.

6. Implantat gemäß einem der Ansprüche 1 oder 2, **dadurch gekennzeichnet, dass** der geschlossene Umriss der Fläche zum dynamischen Zusammenwirken mit der Phalanx (P) ein im Wesentlichen auf der proximodistalen Achse (Z-Z) zentriertes Ellipsoid ist.

7. Implantat gemäß irgendeinem der vorhergehenden Ansprüche, **dadurch gekennzeichnet, dass** das konkave Profil (P62, P162) der Fläche (62, 162) zum dynamischen Zusammenwirken mit der Phalanx (P) im Wesentlichen kreisförmig ist.

8. Implantat gemäß irgendeinem der vorhergehenden Ansprüche, **dadurch gekennzeichnet, dass** die konkave Fläche (41) für die Gelenkverbindung mit dem zugeordneten Knochen (M) eine Kugelkalotte ist, die auf der proximodistalen Achse (Z-Z) zentriert ist.

9. Implantat gemäß irgendeinem der Ansprüche 1 bis 7,
**dadurch gekennzeichnet, dass** die konkave Fläche (141) für die Gelenkverbindung mit dem zugeordneten Knochen (M) im Schnitt in einer axialen dorsoplantaren oder dorsopalmaren Ebene einen Krümmungsradius (RDP) aufweist, der kleiner als ihr Krümmungsradius (RML) in einer mediolateralen axialen Ebene ist.

10. Implantat gemäß irgendeinem der vorhergehenden Ansprüche, **dadurch gekennzeichnet, dass** der Körper (102) auf seiner proximalen Seite (104) eine Aussparung (109) zum Aufnehmen eines Knochenkamms des zugeordneten Knochens, insbesondere des Sesambeinkamms (MC) des Metatarsus (M) aufweist, wobei die Aussparung in einen plantaren oder palmaren Abschnitt des Umfangsrandes (108) des Körpers einmündet.

11. Implantat gemäß irgendeinem der vorhergehenden Ansprüche, **dadurch gekennzeichnet, dass** das Implantat (1, 101) ausschließlich aus dem Körper (2, 102) gebildet ist, der aus einem einzigen Stück realisiert ist.

## Claims

1. Joint implant (1; 101) for interpositioning between a phalange (P) and an associated bone, selected from a metatarsal and a metacarpal, of a metatarsophalangeal joint or a metacarpophalangeal joint,
comprising a body (2, 102) which has, opposite one another on a proximo-distal axis (Z-Z), proximal (4; 104) and distal (6; 106) faces arranged to face, respectively, the associated bone (M) and the phalange (P), the body delimiting, on its proximal face, a concave surface (41; 141) of articulation with the associated bone, **characterised in that** the distal face (6; 106) of the body (2; 102) has a generally pointed shape delimiting a surface (62; 162) of dynamic co-operation with the phalange (P) which extends completely around the proximo-distal axis (Z-Z) and which has, in a section perpendicular to the proximo-distal axis, a closed continuously curved contour (C62; C162) and which has, in axial section, a concave profile (P62; P162).

2. Implant according to claim 1, **characterised in that** the body (2; 102) has, on the proximo-distal axis (Z-Z), a distal extremity at which there is located a vertex surface (61; 161) which is delimited by a region, centred on the proximo-distal axis, of the distal face (6; 106) and starting from which there extends the surface (62; 162) of dynamic co-operation with the phalange (P).

3. Implant according to one of claims 1 or 2, **characterised in that** said closed contour (C62; C162) of the surface (62; 162) of dynamic co-operation with the phalange (P) is a circle centred on the proximo-distal axis (Z-Z).

4. Implant according to claim 3, **characterised in that** the surface (62; 162) of dynamic co-operation with the phalange (P) is a surface of revolution which is centred on the proximo-distal axis (Z-Z) and the generatrix of which corresponds to a segment of the concave profile (P62; P162).

5. Implant according to any one of the preceding claims, **characterised in that**, on its distal face (106), the body (102) delimits at least one surface (163, 164) for seating on the phalange (P), which connects a portion only of the dynamic co-operation surface (162), especially its medial portion or its lateral portion, to the peripheral rim (108) of the body by extending in prolongation of the dynamic surface.

6. Implant according to one of claims 1 or 2, **characterised in that** said closed contour of the surface of dynamic co-operation with the phalange (P) is an ellipsoid substantially centred on the proximo-distal axis (Z-Z).

7. Implant according to any one of the preceding claims, **characterised in that** said concave profile (P62; P162) of the surface (62; 162) of dynamic co-operation with the phalange (P) is substantially circular.

8. Implant according to any one of the preceding claims, **characterised in that** the concave surface (41) of articulation with the associated bone (M) is a spherical cup centred on the proximo-distal axis (Z-Z).

9. Implant according to any one of claims 1 to 7, **characterised in that** the concave surface (141) of articulation with the associated bone (M) has, in a section in an axial dorsoplantar or dorsopalmar plane, a radius of curvature (RDP) which is less than its radius of curvature (RML) in an axial mediolateral plane.

10. Implant according to any one of the preceding claims, **characterised in that** the body (102) delimits, on its proximal face (104), a recess (109) for accommodating a bone crest of the associated bone, especially the sesamoid crest (MC) of the metatarsal (M), which recess is open to a plantar or palmar portion of the peripheral rim (108) of the body.

11. Implant according to any one of the preceding claims, **characterised in that** the implant (1; 101) is composed exclusively of the body (2; 102) made of a single piece.
